# EUROPEAN PATENT APPLICATION

(11) **EP 4 516 289 A1**
(43) Date of publication of application: **05.03.2025**
(21) Application number: 23194385.3
(22) Date of filing: 30.08.2023
(51) Int. Cl.: A61K 9/107, A61K 31/4965, A61P 31/14, A61K 47/18, A61K 47/26

(54) **ANTIVIRAL NASAL SPRAY COMPRISING FAVIPIRAVIR**

(71) Applicant: SPH Sine Pharmaceutical Laboratories Co., Ltd, Shanghai (CN)
(72) Inventor: Mao, YiBin, Shanghai, 201206 (CN)
(74) Representative: Bogensberger, Burkhard

(57) **Abstract**

The invention relates to an antiviral composition for nasal delivery comprising favipiravir dissolved in an aqueous solvent which comprises an aqueous buffer adjusted to a pH within a range of from pH 4 to pH 6, at least one saponin component selected from the group consisting of escin and glycyrrhizin at or above a critical micelle concentration, dexpanthenol at a concentration within a range of from 0.5 to 5 %wt, and favipiravir as an antiviral agent at a concentration of at least 5 or 6 or 8 or 10 mg/ml, wherein the composition is preferably free of an organic solvent.

The invention further relates to said antiviral composition when used in the treatment of viral infections of the airways or the respiratory tract.

## Description

### FIELD

The present application relates to an aqueous composition comprising favipiravir dissolved therein at an increased concentration and having improved storage stability, for use as a nasal spray in the prophylaxis or treatment of viral infections of the upper respiratory tract.

### INTRODUCTION

Favipiravir (FPV) is a member of the class of pyrazines, more specifically a pyrazinecarboxamide derivative. It is a well-known anti-viral agent that inhibits RNA-dependent RNA polymerase of several RNA viruses. In 2014, FPV was approved in Japan to treat cases of influenza that were non-responsive to conventional treatment. Given its efficacy at targeting several strains of influenza, it has been investigated in other countries to treat novel viruses including Ebola and most recently, COVID-19. Favipiravir is slightly soluble in water.

Basically, nasal sprays are broadly accepted by patients suffering from viral or bacterial infections of the airways and the upper respiratory tract in general, particularly in the treatment of running or blocked nose.

During the COVID-19 pandemic favipiravir-based aqueous compositions adapted as nasal sprays turned out to be ineffective due to insufficient bioavailability of the drug upon delivery to nasal mucosa. In the alternative, many individuals received systemic favipiravir medication as a prophylactic or therapeutic treatment. However, some of them developed serious side effects such as glowing eyes and colored finger nails.

It is therefore a goal of the present invention to provide a nasal spray wherein the antiviral drug FPV is dissolved in an acceptable aqueous solvent at an increased concentration sufficiently high for raising the drug's bioavailability to a pharmacologically relevant level upon application to mucosal tissue of the nose and adjacent parts of the upper respiratory tract.

WO201 7009480 discloses a method of improving the solubility of poorly water-soluble hydrophobic drugs in aqueous solutions. The method comprises dissolving the hydrophobic drug in a suitable organic solvent and mixing the organic solvent with an aqueous buffer solution comprising a saponin component selected from the group of escin, glycyrrhizin and Quillaja saponaria extract, along with dexpanthenol and optionally further additives.

While this method has proven quite successful for the solubilization of many drugs for dermatological applications to the outer skin it may not be perfectly suited for providing improved aqueous pharmaceutical compositions for mucosal applications. The reason being that some of them may not sufficiently meet the requirements of mucosal delivery in various cases. More specifically, the effective aqueous concentration of the hydrophobic drug may be far from optimal for the intended purpose, and/or the viscosity of the final solution is too high for sterile filtration and possibly also too high for a convenient administration, i.e. for spraying very small droplets, into the nose. Also, highly viscous compositions may create a sticky feeling in a recipient's nose which is usually regarded as uncomfortable. And, finally, many of these preparations usually encounter stability problems because many hydrophobic drugs tend to precipitate after prolonged storage in aqueous solutions.

It is known that mucosal tissues are extremely sensitive and undesired side effects readily occur even with otherwise well tolerated compounds. For example, even the administration of pure water into the nose can cause sneezing and symptoms of a cold. Hence, there are limited options for developing aqueous formulations of water-insoluble or poorly water-soluble compounds that would be suitable for mucosal administration.

It is therefore an objective of the present invention to overcome the drawbacks known in the art and to provide an improved antiviral aqueous pharmaceutical composition suitable for nasal delivery which comprises favipiravir at a concentration substantially higher than in pure water, and which goes with a substantially increased bioavailability of the antiviral drug in mucosal tissue.

### DESCRIPTION OF THE INVENTION

Favipiravir, hereinafter abbreviated FPV, is a member of the class of pyrazines, i.e. a pyrazinecarboxamide derivative, also known under its IUPAC name 6-fluoro-3-hydroxypyrazine-2-carboxamide (see formula I hereinafter).

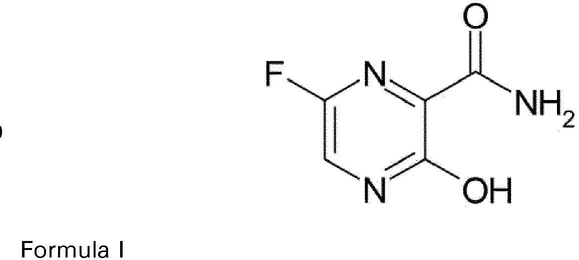

It is an anti-viral active agent that inhibits RNA-dependent RNA polymerase of several RNA viruses. In 2014, FPV was approved in Japan to treat cases of influenza that were non-responsive to conventional treatment. Given its efficacy at targeting several strains of influenza favipiravir has been additionally investigated by various research groups for a possible antiviral activity in the treatment of infections caused by viruses other than influenza viruses, including Ebola virus and, most recently, pandemic coronavirus strains.

From WO201 7009480 it is known that water-insoluble or slightly soluble hydrophobic drugs can be successfully solubilized in aqueous solvents by dissolving the drug in a suitable organic solvent and combining this drug/organic solvent-mixture with a buffered aqueous solvent comprising low concentrations of a saponin compound such as escin and/or glycyrrhizin, and dexpanthenol. The saponin concentrations in the aqueous solvent are chosen such as to allow for the formation of micelles to which the hydrophobic drugs adhere by noncovalent attachment or entrapment.

WO201 7009480 further teaches that the compositions containing dexpanthenol are stable at room temperature for at least one month, many of them even for at least 3 months, i.e., they do not disintegrate into multi-phase systems such as, e.g., liquid-liquid (fatty/aqueous) or liquid-solid (particulate/ aqueous) phases and/or do not lose more than 5% of pharmaceutical or physiological activity during such a storage period. Typically, they are clear, transparent solutions that can be sterile filtered using conventional methods but may also be formulated into non-transparent preparations such as hydrocolloids, emulsions, suspensions, creams, gels or ointments, for specific applications.

Commercially available FPV is reported to be slightly soluble in water. In order to determine the maximum solubility of FPV in water various samples of either raw or micronized FPV powder have been prepared. The maximum solubility in distilled water at a temperature of 25 °C has been determined as 4.27 mg/ml for the micronized powder and as 4.56 mg/ml for the raw FPV powder (see Table 2 hereinafter, samples nos. 22027 and 22028).

In order to check for a possible improvement of FPV solubility in an aqueous solvent, it was decided to follow the protocol of WO2017009480. As a first task it was necessary to find a suitable organic solvent for pre-dissolution of FPV prior to adding the pre-dissolved FPV to the buffered aqueous solvent. The results are depicted in Table 1

**Table 1: Solubility of micronized FPV in different organic solvents**

| **Solvent** | **Visual Inspection** | |
|---|---|---|
| | **Conc. (mg/ml)** | **Comments** |
| PEG 400 | 90.00 | Soluble at 90.00 mg/ml |
| Glycerol | < 33.00 | Not soluble at 33.33 mg/ml |
| PPG 400 | 25.00 | Soluble at 25.00 mg/ml |
| PG | < 10.00 | Not soluble at 10.00 mg/ml |
| AD | 5.00 | Soluble at 5.00 mg/ml |

| | | |
|---|---|---|
| PEG = polyethylene glycol; PPG = polypropylene glycol; PG = propylene glycol AD = distilled water (aqua destillata) | | |

As can be taken from Table 1, the best results were achieved with PEG 400. Accordingly, stock solutions of FPV in PEG 400 were prepared for use in subsequent solubility and storage stability tests, and specifically, for adjusting desired starting concentrations of FPV in the experimental sample preparations.

### EXAMPLE 1: Storage stability of different aqueous formulations of 5 mg/ml FPV

In this experimental set-up, the FPV starting concentration was adjusted to a calculated value of 5 mg/ml. After admixture of all ingredients the experimental samples contained 10 %v/v PEG 400, 2 %wt dexpanthenol, and either a citrate or a phosphate buffer, or a combination of both, adjusted to pH 5.5. The selected saponins were tested each in three concentrations, i.e. escin: 0.10, 0.25 and 0.50% w/v, and glycyrrhizin: 1.0, 2.5 and 5.0% w/v. Glycyrrhizin was applied in the form of dipotassium glycyrrhizinate in this and all other examples.

For the quantification of FPV contents in the experimental sample formulations, an RP-HPLC method using a Zorbax Eclipse Plus C18 column from Agilent Technologies was applied and adapted for the present purpose. The results of the storage stability test of the various FPV containing samples are depicted in Table 2.

**Table 2: Storage stability of different aqueous formulations of 5 mg/ml FPV**

| **Sample No.** | **FPV** | **PEG400 (% v/v)** | **Buffer pH 5.5** | **Dex (% w/v)** | **Escin (% w/v)** | **Glyc (% w/v)** | **FPV d0 mg/ml** | **SI d7 % of d0** |
|---|---|---|---|---|---|---|---|---|
| 22001 | M | 10% | 0.25x citrate | 2 | 0.10 | 0.0 | 4.95 | 97.14 |
| 22002 | | | | 2 | 0.25 | 0.0 | 4.88 | 96.40 |
| 22003 | | | | 2 | 0.50 | 0.0 | 4.86 | 96.88 |
| 22004 | | | | 2 | 0.00 | 1.0 | 4.87 | 100.29 |
| 22005 | | | | 2 | 0.00 | 2.5 | 4.99 | 94.82 |
| 22006 | | | | 2 | 0.00 | 5.0 | 5.01 | 96.61 |
| 22007 | R | | | 2 | 0.10 | 0.0 | 4.87 | 97.48 |
| 22008 | | | | 2 | 0.25 | 0.0 | 4.95 | 95.51 |
| 22009 | | | | 2 | 0.50 | 0.0 | 4.87 | 98.47 |
| 22010 | | | | 2 | 0.00 | 1.0 | 4.83 | 97.71 |
| 22011 | | | | 2 | 0.00 | 2.5 | 4.88 | 99.18 |
| 22012 | | | | 2 | 0.00 | 5.0 | 4.98 | 95.33 |
| 22013 | - | | | 2 | 0.50 | 5.0 | - | - |
| 22014 | M | | 1 x Mc Ilvaine | 2 | 0.10 | 0.0 | 4.95 | 96.07 |
| 22015 | | | | 2 | 0.25 | 0.0 | 5.01 | 93.96 |
| 22016 | | | | 2 | 0.50 | 0.0 | 4.98 | 96.10 |
| 22017 | | | | 2 | 0.00 | 1.0 | 4.89 | 98.44 |
| 22018 | | | | 2 | 0.00 | 2.5 | 4.98 | 94.31 |
| 22019 | | | | 2 | 0.00 | 5.0 | 4.94 | 95.11 |
| 22020 | R | | | 2 | 0.10 | 0.0 | 4.87 | 98.27 |
| 22021 | | | | 2 | 0.25 | 0.0 | 4.92 | 97.82 |
| 22022 | | | | 2 | 0.50 | 0.0 | 4.88 | 96.41 |
| 22023 | | | | 2 | 0.00 | 1.0 | 5.02 | 94.51 |
| 22024 | | | | 2 | 0.00 | 2.5 | 4.88 | 95.95 |
| 22025 | | | | 2 | 0.00 | 5.0 | 4.96 | 95.97 |
| 22026 | - | | | 2 | 0.50 | 5.0 | - | - |
| 22027 | M | - | - | - | - | - | 4.27 | 84.93 |
| 22028 | R | - | - | - | - | - | 4.56 | 80.81 |
| 22029 | M | 10% | 0.25x citrate | - | - | - | 4.96 | 94.62 |
| 22030 | R | | | - | - | - | 4.91 | 96.33 |
| 22031 | M | | 1x Mc Ilvaine | - | - | - | 4.86 | 97.06 |
| 22032 | R | | | - | - | - | 4.89 | 98.30 |

| | | | | | | | | |
|---|---|---|---|---|---|---|---|---|
| M = micronized; R = raw powder; Dexpant = dexpanthenol; Glyc = glycyrrhizin; FPV d0 = HPLC-determined FPV concentration on day 0; SI d7 % of d0 = Stability Index expressed in % of FPV conc. on day 7 relative to FPV conc. on day 0. | | | | | | | | |

When dissolved solely in water (sample numbers 22027, 22028) the pH value dropped to pH 3.5 - 3.6, the maximum FPV solubility was only 4.27 mg/ml (micronized) and 4.56 mg/ml (raw), and the solutions did not remain stable over the observation period of 7 days. Indeed, precipitation of part of the dissolved material occurred and the formation of crystals was visibly detectable. The concentration of dissolved FPV dropped by 15% and 19%, respectively, as determined by HPLC on day 7, i.e. yielding only 85% (micronized) or 81% (raw) of the real, i.e. HPLC analyzed, FPV starting concentrations.

As concerns a possible influence of the two buffer systems and/or of the two FPV qualities i.e. raw vs. micronized, on the solubility and/or storage stability of FPV in aqueous solution at the given FPV concentrations, it can be taken from the results that noticeable differences were not observed.

### EXAMPLE 2: Storage stability of different formulations of 9.5 mg/ml FPV

In an attempt to raise the starting concentration of FPV in a pharmaceutically acceptable aqueous solvent the PEG 400 / FPV stock solution was applied in an amount such as to increase the theoretical / calculated FPV concentration from 5 to about 10mg/ml, more precisely to 9.5mg/ml. After admixture of the predetermined portion of PEG 400 solvent comprising FPV to the aqueous buffer solution the resulting samples contained 10.5 %v/v PEG 400, and 2% dexpanthenol in phosphate buffer pH 5.5. The selected saponins were applied in the following concentrations: escin: 0.01 and 0.05 %w/v; glycyrrhizin: 1.0, 2.5 and 5.0 %w/v. The sample formulations were analyzed by HPLC on day 0 and day 7, the results being depicted in Table 3.

**Table 3: Storage stability of different aqueous formulations of 9.5 mg/ml FPV**

| **Sample No.** | **FPV** | **PEG400 (% v/v)** | **Buffer** | **Dex (%w/v)** | **Escin (%w/v)** | **Glyc (%w/v)** | **FPV d0 mg/ml** | **SI d7 % of d0** |
|---|---|---|---|---|---|---|---|---|
| 22048 | M | 10.5 | 1 x Mc Ilvaine pH 5.5 | 2 | 0.05 | - | 9.16 | 100.87 |
| 22049 | | | | 2 | 0.10 | - | 9.64 | 94.57 |
| 22050 | | | | 2 | - | 1.0 | 9.38 | 95.38 |
| 22051 | | | | 2 | - | 2.5 | 9.53 | 93.11 |
| 22052 | | | | 2 | - | 5.0 | 9.72 | 95.90 |
| 22053 | | | | - | - | - | 9.09 | 98.82 |
| 22054 | | | - | - | - | - | 4.42 | 97.48 |

| | | | | | | | | |
|---|---|---|---|---|---|---|---|---|
| M = micronized FPV; Dex = dexpanthenol; Glyc = glycyrrhizin; d0=day 0; SI d7 % of d0 = Stability Index expressed in % of FPV conc. on day 7 relative to FPV conc. on day 0. | | | | | | | | |

A control sample lacking the saponin components and dexpanthenol (i.e. sample no. 22053) and another control sample lacking the saponins, dexpanthenol and buffer (i.e. sample no. 22054) were also prepared.

It can be taken from Table 3 that the sample preparations containing a buffer as well as a saponin component and dexpanthenol not only perfectly dissolved the weighed amount of 9.5 mg/ml FPV but also stably maintained the starting concentration throughout the 7 day observation period. The FPV solubility on day zero was above 95% relative to the purely calculated, theoretical concentration, and the recovery or stability index after seven days was above 93%.

It can also be taken from Table 3, sample 22054, that pre-dissolving FPV in PEG 400 does not improve FPV solubility when added to unbuffered pure water.

Most surprisingly, however, the data also represents that sample preparation no. 22053 which lacks the saponin and dexpanthenol components yielded a comparable level of dissolved FPV in the buffered aqueous solvent comprising PEG 400. It even stably maintained the drug in solution over the full observation period of 7 days.

### EXAMPLE 3: Storage stability of aqueous formulations of 17 mg/ml FPV

In a next step it was tried to even further increase the FPV concentration in the aqueous solvent system. The theoretical FPV starting concentration was adjusted to approximately 17 mg/ml, more precisely to 17.2 mg/ml (raw FPV) and 17.1 mg/ml (micronized FPV), using stock solutions comprising 85.45 mg/ml (raw) and 86.07 mg/ml (micronized) FPV in PEG 400.

The final experimental samples contained 20% PEG 400 and 2% dexpanthenol in phosphate buffer, pH 5.5, along with varying saponin concentrations:
escin 0.10, 0.25 and 0.50 %w/v; glycyrrhizin: 1.0, 2.5 and 5.0 %w/v.

The factual - as opposed to the calculated, theoretical - FPV concentrations were determined from the sample preparations by HPLC on day 0 and day 7. The results are depicted in Table 4.

**Table 4: Storage stability of different aqueous formulations of 17 mg/ml FPV**

| **Sample No.** | **FP V** | **PEG 400 (% v/v)** | **Buffer** | **Dex (% w/v)** | **Escin (% w/v)** | **Glyc (% w/v)** | **FPV d0 mg/ml** | **SI d7 % of d0** |
|---|---|---|---|---|---|---|---|---|
| 22033 | M | 20 | 1x Mc Ilvaine, pH 5.5 | 2 | 0.10 | - | 9.88 | 97.40 |
| 22034 | | | | | 0.25 | | 10.74 | 100.51 |
| 22035 | | | | | 0.50 | | 9.93 | 98.45 |
| 22036 | | | | | - | 1.0 | 10.03 | 105.59 |
| 22037 | | | | | | 2.5 | 11.33 | 104.94 |
| 22038 | | | | | | 5.0 | 9.44 | 103.89 |
| 22039 | R | | | | 0.10 | - | 9.55 | 102.86 |
| 22040 | | | | | 0.25 | | 9.01 | 102.02 |
| 22041 | | | | | 0.50 | | 8.79 | 101.76 |
| 22042 | | | | | - | 1.0 | 9.85 | 101.80 |
| 22043 | | | | | | 2.5 | 10.78 | 100.67 |
| 22044 | | | | | | 5.0 | 11.54 | 101.22 |
| 22045 | | | | | 0.50 | 5.0 | - | - |
| 22046 | M | | | - | - | - | 11.68 | 96.61 |
| 22047 | R | | | | | | 10.50 | 103.46 |

| | | | | | | | | |
|---|---|---|---|---|---|---|---|---|
| M = micronized FPV; Dex = dexpanthenol; Glyc = glycyrrhizin; d0=day 0; SI d7 % of d0 = Stability Index expressed in % of FPV conc. on day 7 relative to FPV conc. on day 0. | | | | | | | | |

Contrary to expectations the calculated starting concentrations of 17.2 mg/ml (micronized) and 17.1 mg/ml (raw) FPV could not be analytically confirmed in any of the sample preparations. The highest starting concentrations of dissolved FPV determined by HLPC on day 0 were 11.7 mg/ml for micronized FPV in phosphate buffer without saponins and without dexpanthenol, and 11.5 mg/ml for raw FPV in phosphate buffer pH 5.5 and including 5 %w/v glycyrrhizin, confirming the surprising finding disclosed in Example 2. Overall, the storage stability over the observation period of seven days was above 96.6% for all analyzed samples.

### EXAMPLE 4: Influence of PEG 400 on the solubility and storage stability of FPV in aqueous sample preparations containing approximately 10 mg/ml FPV

In yet another experimental set-up a possible influence of the organic solvent PEG 400 on the solubility and stability of buffered aqueous sample preparations containing approximately 10 mg/ml FPV was investigated. PEG 400 was used at concentrations of 0, 2.5, 5.0, 7.5 and 10.0 %v/v. The samples were prepared in phosphate buffer, pH 5.5 with 2% dexpanthenol and including at least one of the saponins in the following concentrations: escin 0.01 and 0.05 %w/v; glycyrrhizin 1.0, 2.5 and 5.0 %w/v. As a control, the solubility and storage stability of FPV solely in phosphate buffer, pH 5.5, i.e. without any other ingredients, was also investigated.

The FPV contents in the sample preparations were analyzed by HPLC on day 0 and day 7, and the results are depicted in Table 5. In this experiment, only micronized FPV powder was used and was directly added to the aqueous formulations in an amount such as to yield a calculated (theoretical) concentration of 10 mg/ml.

**Table 5: Influence of PEG 400 on solubility and stability sample preparations containing approximately 10 mg/ml FPV**

| **Sample No.** | **FPV** | **Buffer** | **PEG 400 (% v/v)** | **Dex (% w/v)** | **Escin (% w/v)** | **Glyc (% w/v)** | **FPV d0 mg/ml** | **SI d7 % of d0** |
|---|---|---|---|---|---|---|---|---|
| 22062 | M | 1x Mc Ilvaine pH 5.5 | - | 2 | 0.05 | - | 10.20 | 102.23 |
| 22063 | | | - | 2 | 0.10 | - | 9.76 | 104.55 |
| 22064 | | | - | 2 | - | 1.0 | 10.46 | 77.58 |
| 22065 | | | - | 2 | - | 2.5 | 10.84 | 93.35 |
| 22066 | | | - | 2 | - | 5.0 | 10.30 | 99.34 |
| 22067 | | | 2.5 | 2 | 0.05 | - | 10.57 | 99.29 |
| 22068 | | | 2.5 | 2 | 0.10 | - | 10.44 | 98.88 |
| 22069 | | | 2.5 | 2 | - | 1.0 | 10.56 | 97.65 |
| 22070 | | | 2.5 | 2 | - | 2.5 | 10.69 | 95.44 |
| 22071 | | | 2.5 | 2 | - | 5.0 | 10.44 | 100.78 |
| 22072 | M | 1x Mc Ilvaine pH 5.5 | 5.0 | 2 | 0.05 | - | 10.77 | 95.88 |
| 22073 | | | 5.0 | 2 | 0.10 | - | 11.03 | 94.88 |
| 22074 | | | 5.0 | 2 | - | 1.0 | 10.87 | 89.14 |
| 22075 | | | 5.0 | 2 | - | 2.5 | 10.97 | 94.54 |
| 22076 | | | 5.0 | 2 | - | 5.0 | 10.68 | 96.06 |
| 22077 | | | 7.5 | 2 | 0.05 | - | 10.82 | 94.61 |
| 22078 | | | 7.5 | 2 | 0.10 | - | 10.79 | 96.42 |
| 22079 | | | 7.5 | 2 | - | 1.0 | 11.12 | 90.00 |
| 22080 | | | 7.5 | 2 | - | 2.5 | 10.67 | 97.28 |
| 22081 | | | 7.5 | 2 | - | 5.0 | 10.70 | 94.18 |
| 22082 | | | 10.0 | 2 | 0.05 | - | 10.40 | 99.39 |
| 22083 | | | 10.0 | 2 | 0.10 | - | 10.02 | 96.56 |
| 22084 | | | 10.0 | 2 | - | 1.0 | 10.36 | 97.28 |
| 22085 | | | 10.0 | 2 | - | 2.5 | 10.77 | 88.04 |
| 22086 | | | 10.0 | 2 | - | 5.0 | 10.48 | 97.16 |
| 22087 | | | - | - | - | - | 10.60 | 71.14 |

| | | | | | | | | |
|---|---|---|---|---|---|---|---|---|
| M = micronized FPV; Dex = dexpanthenol; Glyc = glycyrrhizin; d0=day 0; SI d7 % of d0 = Stability Index expressed in % of FPV conc. on day 7 relative to FPV conc. on day 0. | | | | | | | | |

In all samples the concentration of actually dissolved FPV on day zero was at or above 98% of the calculated, theoretical concentration.

Also, except for samples 22064, 22074, 22079 and 22085 the recovery rate as an indicator of storage stability (e.g. stability index) was above 94% after seven days of storage at 25°C. Formulations 22064 and 22074, both containing 1% glycyrrhizin contained visible crystals on day seven. When FPV was dissolved in phosphate buffer pH 5.5 only (sample 22087), i.e. in the absence of saponin, dexpanthenol and organic solvent (e.g. PEG 400), the actual starting concentration determined on day 0 was 10.6 mg/ml. However, this formulation was not stable over the seven days observation period. The dissolved FPV concentration dropped from 10.6mg/ml to 7.54 mg/ml on day 7. This was also confirmed by the presence of a big crystal on day 7.

**Table 6: Comparison of selected experimental FPV samples**

| **Sample No.** | **calc. FPV mg/ml** | **FPV** | **PEG400 (% v/v)** | **Buffer** | **Dex (%w/v)** | **Escin (%w/v)** | **Glyc (%w/v)** | **FPV d0 mg/ml** | **SI d7 % of d0** |
|---|---|---|---|---|---|---|---|---|---|
| 22027 | 5 | M | - | - | - | - | - | 4.27 | 84.93 |
| 22028 | 5 | R | - | - | - | - | - | 4.56 | 80.81 |
| 22087 | 10 | M | - | 1xMc | - | - | - | 10.60 | 71.14 |
| 22064 | 10 | M | - | 1xMc | 2 | - | 1.0 | 10.46 | 77.58 |
| 22062 | 10 | M | - | 1xMc | 2 | 0.05 | - | 10.20 | 102.23 |
| 22066 | 10 | M | - | 1xMc | 2 | - | 5.0 | 10.30 | 99.34 |
| 22054 | 9.5 | M | 10.5 | - | - | - | - | 4.42 | 97.48 |
| 22053 | 9.5 | M | 10.5 | 1xMc | - | - | - | 9.09 | 98.82 |
| 22046 | 17 | M | 20 | 1xMc | - | - | - | 11.68 | 96.61 |
| 22047 | 17 | R | 20 | 1xMc | - | - | - | 10.50 | 103.46 |
| 22074 | 10 | M | 5.0 | 1xMc | 2 | - | 1.0 | 10.87 | 89.14 |
| 22079 | 10 | M | 7.5 | 1xMc | 2 | - | 1.0 | 11.12 | 90.00 |
| 22085 | 10 | M | 10.0 | 1xMc | 2 | - | 2.5 | 10.77 | 88.04 |
| 22037 | 17 | M | 20 | 1xMc | 2 | - | 2.5 | 11.33 | 104.94 |
| 22044 | 17 | R | 20 | 1xMc | 2 | - | 5.0 | 11.54 | 101.22 |

Summarizing the above and referring to Table 6 it can be concluded that:
a) Without buffer the maximum solubility of FPV in an aqueous solution remains below 5 mg/ml, regardless of the amount of FPV added. This is not an option of the present invention.
b) The omission of the organic solvent PEG 400 can be successfully compensated by providing a suitable buffer adjusted to a pH value within a range of from 4 - 6, for example pH 5.0 or 5.5, supplemented with 0.5 - 5 % v/v dexpanthenol, e.g. 2% dexpanthenol, and either 0.01-1 %w/v of escin, e.g. 0.03 or 0.05% w/v escin, or 2.5 - 5% w/v, e.g. 5% w/v, of glycyrrhizin.
c) The presence of low concentrations, e.g. less than 2.5%, of glycyrrhizin in buffered aqueous samples comprising no or only low amounts of PEG 400, e.g. 10 %v/v PEG400 or less, seems to impair shelf life or storage stability of FPV, whereas - unexpectedly - at PEG400 concentrations of more than 10 %v/v, e.g. 20 %v/v PEG400, this negative effect seems to vanish. Also unexpectedly, a similar "curative" effect was observed with experimental samples comprising more than 2.5% w/v glycyrrhizin, e.g. 5% w/v, wherein despite the absence of an organic solvent such as PEG400 the shelf life of FPV in buffered aqueous solutions was not impaired over the observance period of 7 days.

### EXAMPLE 5: Bioavailability test

Bioavailability is a key pharmacokinetic property that affects the ability of a drug to reach systemic circulation unaltered after administration. It is dependent on multiple factors, both physiological and drug related, such as solubility, pH, absorption area, permeability, and metabolism, as well as the route of administration. Successfully increasing the solubility of a poorly water-soluble drug may therefore be an important step towards increased bioavailability but is not per se indicative or predictive of an improvement in bioavailability. Even after decades of drug development it is still an ongoing challenge to meet all requirements for rendering an active pharmaceutical ingredient (API) of low solubility commercially successful, including in particular enhanced solubilization, enhanced bioavailability and good tolerability (absence of undesired side-effects). The art is replete with literature on these important issues.

For testing the bioavailability of favipiravir from aqueous formulations, ex-vivo penetration tests were conducted. More specifically, aqueous solutions comprising favipiravir at concentrations of about 5 mg/ml and about 10 mg/ml were prepared in accordance with the previous examples, and drug penetration into excised porcine nasal mucosa was measured.

FPV was provided by NasPress Pharma (China). Three different formulations were prepared at a nominal FPV concentration of 10 mg/ml:
i) FPV in water, adjusted to pH 5.0;
ii) FPV in citrate buffer pH 5.0; and
iii) FPV in citrate buffer pH 5.0, plus 0.03% w/v escin, plus 2% v/v dexpanthenol.

For the ex-vivo experiment, 0.5 *µ*L of each formulation was applied per 1 mg tissue to the apical surface of the porcine nasal mucosa. The samples were incubated for 15 min in a cell culture incubator (37 °C, 5% CO2, 100% relative humidity). The trial comprised five replicates per condition and per formulation. FPV that permeated the tissue was quantified via liquid chromatography tandem mass spectrometry (LC-MS/MS) and the analyses were performed by a Contract Research Organization (CRO).

Fresh porcine nasal mucosa was obtained from a euthanized pig (German large white). Uniform parts of the nasal mucosa were taken with a biopsy punch and were stored in Ringer's solution at room temperature. Prior to treatment, liquid was drained off and the nasal mucosa tissue pieces were weighed. Nasal mucosa pieces were placed apical side up into 48-well cell culture plates and 0.5 *µ*L/1 mg tissue of the respective formulation was applied onto the mucosal surface

Treated samples were incubated for 15 min in a cell culture incubator (37 °C, 5% CO2, 100% relative humidity). At the end of the incubation time, the mucosa was washed with Ringer solution. Individual mucosa samples were shock-frozen in liquid nitrogen and shipped to the CRO for LC-MS/MS analysis to quantify the content of FPV that permeated the mucosa.

The real starting concentrations of solubilized FPV in each formulation were analyzed by RP-HPLC. The nominal starting concentration of 10 mg/ml FPV was only reached in formulation (iii) containing citrate buffer + saponin + dexpanthenol (i.e. FPV 9.8 mg/ml). In the buffered formulation (ii) lacking the saponin and dexpanthenol contents the FPV concentration was determined as 6.5 mg/ml, and in the purely aqueous formulation (i) the FPV concentration was 6.0 mg/ml, respectively. The pH and visual inspection were also recorded and are represented in Table 7.

**Table 7: pH measurement and visual inspection of FPV formulations (i) - (iii)**

| Sample Number | pH d0 | Visual Inspection on day 0 (d0) |
|---|---|---|
| (i) 1007_23011 | 5.09 | FPV was not fully dissolved, precipitation + |
| (ii) 1007_23012 | 5.08 | precipitation |
| (iii) 1007_23013 | 4.99 | clear |

The visual appearance of the above FPV formulations one hour after compounding revealed that the only clear formulation was formulation (iii) containing buffer + saponin + dexpanthenol (i.e. 1007_23013). Formulation (ii) in buffer (1007_23012), and formulation (i) in water (1007_23011) were not clear.

The evaluation of the porcine nasal mucosa pieces treated as disclosed above revealed that the best bioavailability of FPV was determined with formulation (iii), i.e. comprising citrate buffer pH 5.0, along with 0.03% wt escin and 2%v/v dexpanthenol. More specifically, while FPV penetration from formulation (iii) into the ex-vivo porcine nasal mucosa reached a level of 650 *µ*g FPV per gram mucosal tissue, the corresponding values for the buffered formulation (ii) and the purely aqueous formulation (i) were determined as 406 *µ*g/g, and 299 *µ*g/g, respectively.

From the above it can be taken that the solubility of favipiravir in an aqueous solution can be successfully increased from previously known approximately 4.5 mg/ml in pure water up to more than 10 mg/ml by ensuring a stable pH within a range of from pH 4 to 6, typically pH 5.0 - 5.5, using a phosphate or citrate buffer system, and optionally adding a saponin component such as escin, preferably alpha-escin, at a concentration of from 0.01 - 1 %w/v, or glycyrrhizin at a concentration of from 2.5 - 5% w/v, and alternatively or in addition adding 1 to 5 % v/v dexpanthenol. Even more so, adding dexpanthenol and a saponin component seems to keep the aqueous buffered FPV formulations stable during storage at room temperature, at least for an observance period of 7 days.

Also, pre-dissolution of FPV in an organic solvent in accordance with the solubilization method disclosed in WO 2017009480 is not necessary for achieving increased concentrations of dissolved or solubilized FPV and can thus be waived, which renders the resulting buffered aqueous FPV solutions even better suitable for mucosal delivery.

And finally, the buffered aqueous FPV formulations go with increased bioavailability as evidenced by an ex-vivo permeation experiment using porcine nasal mucosa, wherein the buffered aqueous FPV formulations yielded superior penetration results relative to unbuffered, aqueous formulations, with the buffered and saponin + dexpanthenol supplemented formulations (formulation type iii) yielding the best penetration and thus bioavailability results. This has in effect that the buffered FPV formulations and in particular the saponin +dexpanthenol supplemented buffered FPV formulations are able to generate drug levels in the nasal mucosa that are required for a sound antiviral efficacy of FPV when applied as a nasal spray onto the nasal mucosa.

And last but not least, given the specifically selected ingredients, i.e. escin or glycyrrhizin as a saponin component along with dexpanthenol, all of which ingredients well known in the art for safe and beneficial use in pharmaceutical compositions, the present favipiravir formulations are well tolerated by recipients when applied as a spray mist to the nasal mucosa.

The aqueous buffered pharmaceutical compositions comprising FPV prepared in line with the present invention therefore allow for the prophylactic or therapeutic treatment of individuals in need of or benefitting from an antiviral treatment of the airways and the upper respiratory tract.

## Claims

1. An antiviral composition for nasal delivery comprising favipiravir dissolved in an aqueous solvent, **characterized in that** the composition comprises an aqueous buffer adjusted to a pH within a range of from pH 4 to pH 6, at least one saponin component selected from the group consisting of escin and glycyrrhizin at or above a critical micelle concentration, dexpanthenol at a concentration within a range of from 0.5 to 5 % v/v, and favipiravir as an antiviral agent at a concentration of at least 5 mg/ml.

2. The antiviral composition of claim 1, **characterized in that** the aqueous buffer is a citrate buffer or a phosphate buffer, or a combination of both, adjusted to a pH within a range of from pH 4.5 to pH 5.5.

3. The antiviral composition of claim 1 or 2, **characterized in that** it comprises escin, preferably alpha-escin, at a concentration of from 0.01 to 0.5 %w/v, and/or glycyrrhizin at a concentration of from about 2.5 to about 5 % w/v.

4. The antiviral composition of any one of claims 1 to 3, **characterized in that** it comprises dexpanthenol at a concentration of from 1.5 - 2.5 % v/v.

5. The antiviral composition of any one of claims 1 to 4, **characterized in that** it comprises favipiravir solubilized at a concentration of at least 6 mg/ml or at least 8 mg/ml or at least 10 mg/ml.

6. The antiviral composition of any one of claims 1 to 5, **characterized in that** it is free of an organic solvent.

7. The antiviral composition of any one of claims 1 to 6, **characterized in that** it is formulated as a spray for nasal delivery.

8. The antiviral composition of any one of claims 1 to 7, for use in the prophylactic or therapeutic treatment of viral infections of the airways or the respiratory tract.
